# EUROPEAN PATENT SPECIFICATION

(11) **EP 3 447 120 B1**
(45) Date of publication and mention of the grant of the patent: **23.06.2021**
(21) Application number: 17802821.3
(22) Date of filing: 24.05.2017
(51) Int. Cl.: C12M 3/00, C12N 11/08, C12M 1/12, C12M 1/00

(54) **THREE-DIMENSIONAL THIN FILM STRUCTURE HAVING MICROPARTICLES ENCLOSED THEREIN AND METHOD FOR MANUFACTURING SAME**
DREIDIMENSIONALE DÜNNSCHICHTSTRUKTUR MIT DARIN EINGESCHLOSSENEN MIKROPARTIKELN UND HERSTELLUNGSVERFAHREN DAFÜR
STRUCTURE TRIDIMENSIONNELLE À COUCHES MINCES RENFERMANT DES MICROPARTICULES ET SON PROCÉDÉ DE FABRICATION

(30) Priority: 24.05.2016 JP 2016103362
(43) Date of publication of application: 27.02.2019
(73) Proprietor: Nippon Telegraph And Telephone Corporation, Chiyoda-ku, Tokyo 100-8116 (JP)
(72) Inventor: TESHIMA Tetsuhiko, Musashino-shi Tokyo 180-8585 (JP); UENO Yuko, Musashino-shi Tokyo 180-8585 (JP); SASAKI Satoshi, Musashino-shi Tokyo 180-8585 (JP); TSUKADA Shingo, Musashino-shi Tokyo 180-8585 (JP); NAKASHIMA Hiroshi, Musashino-shi Tokyo 180-8585 (JP); GOTO Touichiro, Musashino-shi Tokyo 180-8585 (JP)
(74) Representative: Brevalex
(86) International application number: PCT/JP2017/019302
(87) International publication number: WO 2017/204235

(56) References cited:
- EP-A1- 2 423 162
- JP-A- 2015 029 468
- D I Zakharchenko ET AL: "Encapsulation of particles and cells using stimuli-responsive self-rolling polymer films", , 1 January 2014 (2014-01-01), XP055444733, Retrieved from the Internet: URL:http://www.qucosa.de/fileadmin/data/qu cosa/documents/14140/Svetlana_Zakharchenko _Dissertation.pdf [retrieved on 2018-01-25]
- LUCHNIKOV V ET AL: "Self-Rolled Polymer and Composite Polymer/Metal Micro- and Nanotubes with Patterned Inner Walls", ADVANCED MATERIALS, WILEY-VCH GERMANY, DE, vol. 17, no. 9, 1 May 2005 (2005-05-01), pages 1177-1182, XP002621574, ISSN: 0935-9648, DOI: 10.1002/ADMA.200401836 [retrieved on 2005-04-25]
- VASIEV ISKANDAR ET AL: "Self-folding nano- and micropatterned hydrogel tissue engineering scaffolds by single step photolithographic process", MICROELECTRONIC ENGINEERING, ELSEVIER PUBLISHERS BV., AMSTERDAM, NL, vol. 108, 13 April 2013 (2013-04-13), pages 76-81, XP028563457, ISSN: 0167-9317, DOI: 10.1016/J.MEE.2013.04.003
- VALERIY LUCHNIKOV ET AL: "Self-Rolled Polymer Tubes: Novel Tools for Microfluidics, Microbiology, and Drug-Delivery Systems", MACROMOLECULAR RAPID COMMUNICATIONS, vol. 32, no. 24, 15 December 2011 (2011-12-15), pages 1943-1952, XP055444742, DE ISSN: 1022-1336, DOI: 10.1002/marc.201100482
- ZAKHARCHENKO, SVETLANA: 'Encapsulation of particles and cells using stimuli-responsive self-rolling polymer films' DISSERTATION, DOCTOR RERUM NATURALIUM 2014, pages 1 - 149, XP055444733
- XI, WANG ET AL.: 'Rolled-up functionalized nanomembranes as three-dimensional cavities for single cell studies' NANO LETT. vol. 14, 2014, ISSN 1530-6984 pages 4197 - 4204, XP055444734
- STROGANOV, VLADISLAV ET AL.: 'Biodegradable Self-Folding Polymer Films with Controlled Thermo-Triggered Folding' ADVANCED FUNCTIONAL MATERIALS vol. 24, 2014, ISSN 1616-3028 pages 4357 - 4363, XP055444738
- LUCHNIKOV, VALERIY ET AL.: 'Self-rolled polymer tubes: novel tools for microfluidics, microbiology, and drug-delivery systems' MACROMOL. RAPID COMMUN. vol. 32, 2011, ISSN 1521-3927 pages 1943 - 1952, XP055444742
- HUANG, GAOSHAN ET AL.: 'Rolled-up transparent microtubes as two-dimensionally confined culture scaffolds of individual yeast cells' LAB CHIP vol. 9, 2009, ISSN 1473-0189 pages 263 - 268, XP055444745
- ARAYANARAKOOL, RERNGCHAI ET AL.: 'Tailoring three-dimensional architectures by rolled-up nanotechnology for mimicking microvasculatures' LAB CHIP vol. 15, 2015, ISSN 1473-0189 pages 2981 - 2989, XP055444747
- ZAKHARCHENKO, SVETLANA ET AL.: 'Stimuli- responsive hierarchically self-assembled 3D porous polymer-based structures with aligned pores' J. MATER. CHEM. B vol. 1, 2013, ISSN 2050-7518 pages 1786 - 1793, XP055444749
- ZAKHARCHENKO, SVETLANA: 'Fully Biodegradable Self-Rolled Polymer Tubes: A Candidate for Tissue Engineering Scaffolds' BIOMACROMOLECULES vol. 12, 2011, ISSN 1525-7797 pages 2211 - 2215, XP055346278
- LIU, LU ET AL.: 'Redox-triggered self-rolling robust hydrogel tubes for cell encapsulation' MACROMOL. RAPID COMMUN. vol. 35, 2014, ISSN 1521-3927 pages 344 - 349, XP055444752

## Description

### TECHNICAL FIELD

The present invention relates to a three-dimensional thin film structure in which a microparticle is encapsulated inside a polymer thin film structure and a method for producing the same. In particular, the present invention relates to a tubular structure enabling isolated cultivation and transport operation of an adherent cell by encapsulating the adherent cell and a method for producing the same.

### BACKGROUND ART

Techniques for manipulating cells derived from living tissue at a single cell level are required not only for a fundamental research of cell biology but also for a wide range of fields such as regenerative medicine and drug discovery screening. Techniques for manipulating adherent cells such as epithelial cells, nerve cells, liver cells and the like constituting the tissues in a living body can be applied not only to cell sorting and analysis by a cell sorter, but also to construct pseudo three dimensional biological tissues by assembling cells in vitro. By constructing a pseudo three dimensional biological tissue, it is possible to conduct the dynamic analysis of a target living tissue and a susceptibility test to a drug, and to further prepare a carrier for organ reconstruction and cell transplantation.

In the past, it has been possible to select and recover individual cells of suspended (non-adherent) cells such as blood cells relatively easily by operating techniques of micropipettes, microfluidic devices and the like because of their floating characteristics. On the other hand, because of the property that adherent cells cannot grow unless being adhered to each other or to a culture substrate, it is usual to manipulate them after chemically releasing the cells once with an enzyme such as trypsin, or physically destroy the adhesion between the cells and the substrate to liberate them. However, it has been difficult to observe and analyze the intrinsic activity of the cells because loss of the cell membrane surface marker, disruption of the skeletal system, and cell death are induced by these chemically or physically liberating operations. Therefore, it has been essential to establish an operating method that enables an operation while maintaining the adhesion state of the cells, with lesser damage to the cells.

In recent years, attention has been paid to a technique for manufacturing a minute dynamic substrate onto which adherent cells can be adhered using a microfabrication technique, and culturing and manipulating the cells on the surface thereof (Non-Patent Document 1). Using a self-assembling force to fabricate a tubular structure and adhering cells inside it, it became possible to manipulate in a state where the adhesiveness of cells was maintained. In addition, it became possible to observe the behavior of cells under a three-dimensional environment like a tissue (Non-Patent Document 2). The tubular structure as described above is fabricated using a microfabrication process such as a photolithography technique. Therefore, in general, thin films of inorganic substances such as metals and silicon compounds formed by crystal growth or vapor deposition are used for the material of the substrate and the material of the sacrificial layer used for liberating the substrate. In such a thin film of an inorganic substance, thin film layers composed of plural kinds of elements make up a structure in which they are in close contact with each other in the thin film. Therefore, a stress distribution occurs in the planar film due to the gradient of the lattice constant in the thickness direction, and the thin film is bent to form a three-dimensional shape.

Other examples of manufacturing of self-rolling polymer films can be found in Non-Patent Documents 3 and 4.

### Citation List

[Non-Patent Document 1] B. Radha, M. Arif, R. Datta, T. K. Kundu, G. U. Kulkarni, Nano Research 2010, 3, 738.
[Non-Patent Document 2] W. Xi, C. K. Schmidt, S. Sanchez, D. H. Gracias, R. E. Carazo-Salas, S. P. Jackson, O. G. Schmidt, Nano Letters, 2014, 14, 4197.
[Non-Patent Document 3] D. I. Zakharchenko et al., « Encapsulation of particles and cells using stimuli-responsive self-rolling polymer films », 1 January 2014, XP055444733.
[Non-Patent Document 4] Luchnikov V. et al., « Self-Rolled Polymer and Composite Polymer/Metal Micro- and Nanotubes with Patterned Inner Walls », Advanced Materials, Wiley-VCH Germany, vol. 17, no. 9, 1 May 2005, pages 1177-1182, XP002621574.

### SUMMARY OF INVENTION

### Technical Problem

However, since a metal thin film generally has low biocompatibility, it is difficult to bring cells into contact therewith for a long period of time, and it is not suitable as an adhesive substrate for cells. In addition, since an etching solution with high cytotoxicity is used in the manufacturing process and lift-off process, it is necessary to thoroughly clean the substrate after fabrication of the three-dimensional structure, and after washing, the cells are introduced inside. Therefore, it is difficult to operate in a state where the substrate is isolated. In addition, since introduction of cells into the inside of the tubular structure depends on accidental entry of cells into the inside of the tubular structure, there was a problem that the success rate of cell encapsulation is low.

In view of the above circumstances, an object of the present invention is to provide a three-dimensional thin film structure having a high efficiency of introducing microparticles such as cells and capable of culturing cells or the like in an internal space thereof for a long period of time.

### Solution to Problem

The present invention concerns a three-dimensional structure according to claim 1.

In the three-dimensional structure, the microparticle may be a cell; the three-dimensional structure may further include a layer composed of an extracellular matrix on a surface of the polymer film ; the polymer film may have a thickness of 15 to 400 nm ; the cell may be an adherent cell and be adhered to the polymer film ; the three-dimensional structure may have a biological tissue-like structure, and the cell may form a cell aggregate of a biological tissue-like structure.

The present invention also concerns a biological tissue-like structure according to claim 7.

The present invention also concerns a method for producing a three-dimensional structure encapsulating a microparticle according to claim 8.

The method for producing a three-dimensional structure may further include a step of forming the sacrificial layer on the substrate, wherein the step (b) is a step of adding a suspension containing the microparticle to the polymer film having the plurality layers.

In the method for producing a three-dimensional structure, the microparticle may be a cell; the method for producing a three-dimensional structure may further include a step of forming a layer composed of an extracellular matrix on a surface of the polymer film having the plurality of layers; in the method for producing a three-dimensional structure, the polymer film may have a thickness of 15 to 400 nm.

The present invention also concerns a laminate according to claim 13.

The laminate may further include a layer composed of an extracellular matrix laminated on the polymer film having the plurality of layers.

In the laminate, the polymer film may have a thickness of 15 to 400 nm.

### Advantageous Effects of Invention

According to the present invention, there are provided a three-dimensional thin film structure having a high efficiency of introducing microparticles such as cells and capable of culturing cells and the like in an internal space thereof for a long period of time and a method for manufacturing the same.

### BRIEF DESCRIPTION OF DRAWINGS

FIG. 1 is a perspective view of a three-dimensional structure according to one embodiment of the present invention.
FIG. 2 is a cross-sectional view of a three-dimensional structure according to one embodiment of the present invention.
FIG. 3 is a conceptual diagram of bending by a laminated structure of a polymer thin film having two layers having different swelling ratios.
FIG. 4 is a conceptual diagram showing one embodiment of self-assembly into a three-dimensional shape using a thin film having a two-layer structure and encapsulation of cells.
FIG. 5A is an example of a process diagram of formation of a three-dimensional thin film structure encapsulating microparticles.
FIG. 5B is an example of a process diagram of formation of a three-dimensional thin film structure encapsulating microparticles.
FIG. 5C is an example of a process diagram of formation of a three-dimensional thin film structure encapsulating microparticles.
FIG. 5D is an example of a process diagram of formation of a three-dimensional thin film structure encapsulating microparticles.
FIG. 5E is an example of a process diagram of formation of a three-dimensional thin film structure encapsulating microparticles.
FIG. 5F is an example of a process diagram of formation of a three-dimensional thin film structure encapsulating microparticles.
FIG. 5G is an example of a process diagram of formation of a three-dimensional thin film structure encapsulating microparticles.
FIG. 5H is an example of a process diagram of formation of a three-dimensional thin film structure encapsulating microparticles.
FIG. 5I is an example of a process diagram of formation of a three-dimensional thin film structure encapsulating microparticles.
FIG. 5J is an example of a process diagram of formation of a three-dimensional thin film structure encapsulating microparticles.
FIG. 6A shows an electron microscope image of thin film layers. It is an electron microscope (SEM) image of a thin film pattern formed by using a lithography technique.
FIG. 6B shows an electron microscope image of thin film layers. It is an electron microscope (SEM) image of a thin film pattern formed by using a lithography technique.
FIG. 6C shows an electron microscope image of thin film layers. It is a SEM image of a cross section after cutting thin film layers with a focused ion beam.
FIG. 6D shows a result of energy dispersive X-ray analysis of thin film layers. This is the result of energy dispersive X-ray analysis of each layer of the thin film layers and a substrate 13.
FIG. 7A shows a self-assembly of a rectangular thin film into a tubular structure after addition of an ethylenediamine tetraacetic acid (EDTA) solution. It is a phase contrast microscope image showing a state of self-assembly into a tubular structure. This is the case where there are no cells on the thin film.
FIG. 7B shows a self-assembly of a rectangular thin film into a tubular structure after addition of an ethylenediamine tetraacetic acid (EDTA) solution. It is a phase contrast microscope image showing a state of self-assembly into a tubular structure. This is the case where there are cells on the thin film.
FIG. 7C shows a self-assembly of a rectangular thin film into a tubular structure after addition of an ethylenediamine tetraacetic acid (EDTA) solution. It shows a correlation between the curvature radius of the tubular structure and the thickness of a parylene layer.
FIG. 8A shows a micrograph of a tubular structure encapsulating cells. It is a phase contrast microscope image of a tubular structure encapsulating Chinese hamster ovary-derived (CHO) cells.
FIG. 8B shows a micrograph of a tubular structure encapsulating cells. It is a phase contrast microscope image of a tubular structure encapsulating human embryonic kidney-derived (HEK) cells.
FIG. 8C shows a micrograph of a tubular structure encapsulating cells. It is a confocal microscope image of a tubular structure encapsulating CHO cells.
FIG. 8D shows a micrograph of a tubular structure encapsulating cells. This is an example of producing a biological tissue-like structure having a length in the major axis direction of 1 cm or more.
FIG. 9A shows an example of production of a tubular structure encapsulating primary neurons. It is a phase contrast microscope image at an initial stage of culture of a tubular structure encapsulating primary neurons.
FIG. 9B shows an example of production of a tubular structure encapsulating primary neurons. It is a phase contrast microscope image after long-term culture of a tubular structure encapsulating primary neurons.
FIG. 9C shows an example of production of a tubular structure encapsulating primary neurons. It is a phase contrast microscope image of a tubular structure moved on a substrate.
FIG. 9D shows an example of production of a tubular structure encapsulating primary neurons. It is a SEM image of a tubular structure moved on a substrate.
FIG. 9E shows an example of production of a tubular structure encapsulating primary neurons. It is a confocal microscope image obtained by observing changes in fluorescence intensities of cells inside and outside the tubular structure when cells were stimulated by adding potassium chloride.
FIG. 10A shows an example of production of a tubular structure encapsulating primary cardiac myocytes. It is a phase contrast microscope image of a fibrous structure prepared by culturing a tubular structure encapsulating primary cardiac myocytes.
FIG. 10B shows an example of production of a tubular structure encapsulating primary cardiac myocytes. The upper figures are a phase contrast microscope image (above) of a tubular structure encapsulating primary cardiac myocytes and an image (below) showing the amount of change with respect to the resting state at the time of beating of primary cardiac myocytes. The images were created using an image processing program ImageJ provided by National Institute of Health (NIH). The lower figure is a graph showing the amount of change (intensity) over time detected in the upper figures.
FIG. 10C shows an example of production of a tubular structure encapsulating primary cardiac myocytes. The upper figure is a confocal microscope image obtained by observing changes in fluorescence intensities of cells inside and outside the tubular structure when cells were stimulated by adding potassium chloride. The lower figure is a graph showing changes in the fluorescence intensity over time detected in the upper figure.
FIG. 11A shows an example of fabrication of a three-dimensional structure produced using thin films of various two-dimensional shapes. It is a production example of forming a three-dimensional structure having a spherical holding gripper structure from a thin film having a radial floral pattern shape.
FIG. 11B shows an example of fabrication of a three-dimensional structure produced using thin films of various two-dimensional shapes. It is an example of producing a three-dimensional structure having a T-shaped structure in which only one direction of a cross shape is bent from a thin film having a cross shape.
FIG. 11C is an example of fabrication of a three-dimensional structure produced using thin films of various two-dimensional shapes. It is an example of producing a three-dimensional structure having a three-dimensional human-type structure via an unbending joint portion simulating a human form by joining a cross-shaped thin film to a rectangular thin film.
FIG. 11D is an example of fabrication of a three-dimensional structure produced using thin films of various two-dimensional shapes. It is an example of producing a three-dimensional structure from a thin film in which pores are formed inside.
FIG. 11E is an example of fabrication of a three-dimensional structure produced using thin films of various two-dimensional shapes. It is an example of producing a three-dimensional structure from a human type thin film in which pores are formed inside.
FIG. 11F is an example of fabrication of a three-dimensional structure produced using thin films of various two-dimensional shapes. It is an example of producing a three-dimensional structure having a helical structure from a thin film having a wave-like shape.
FIG. 11G is an example of fabrication of a three-dimensional structure produced using thin films of various two-dimensional shapes. It is an example of producing a three-dimensional structure having a mesh-like net structure is produced from a thin film having a lattice shape.
FIG. 12A is a diagram for explaining the curvature radius ρ of a tubular structure, the thickness tₚ of a parylene layer, the thickness tₛ of a silk fibroin gel layer, the lateral width w of a thin film, and the length I of the thin film in the major axis direction.
FIG. 12B shows a correlation between the curvature radius ρ of the tubular structure and the thickness tₚ of the parylene layer.
FIG. 12C shows a correlation between the curvature radius ρ of the tubular structure and the thickness tₚ of the parylene layer. The black squares show the case where the thickness tₛ of the silk fibroin gel layer is 100 nm and the black circles show the case where the thickness tₛ of the silk fibroin gel layer is 210 nm.
FIG. 12D shows a correlation between the curvature radius ρ of the tubular structure and the lateral width w of the thin film.
FIG. 12E shows the correlation between the curvature radius ρ of the tubular structure and the length I of the thin film in the major axis direction.

### DESCRIPTION OF EMBODIMENTS

### <Three-dimensional structure>

A three-dimensional structure of the present invention is a three-dimensional structure as defined in the claims. Further, in one embodiment, the three-dimensional structure as defined in the claims is a three-dimensional structure composed of a polymer film having a plurality of layers, wherein a microparticle is encapsulated in an internal space of the aforementioned three-dimensional structure, and each layer of the aforementioned polymer film having the plurality of layers has mechanical strengths different from each other. Hereinafter, the three-dimensional structure of the present invention will be described with reference to drawings showing a preferred embodiment of the present invention.

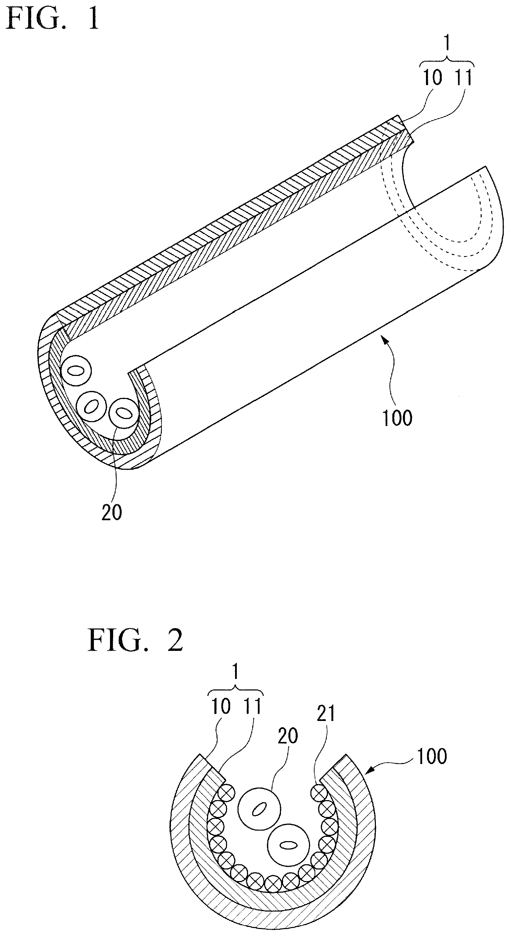

FIG. 1 is a perspective view of a three-dimensional structure according to one embodiment of the present invention, and FIG. 2 is a cross-sectional view of a three-dimensional structure according to one embodiment of the present invention. In the drawings, reference numerals 100, 1, 10, 11, 20 and 21 denote a three-dimensional structure, a thin film, a first thin film layer, a second thin film layer, a microparticle such as a cell, and an adhesion protein, respectively.

As shown in FIGS. 1 and 2, the three-dimensional structure 100 has a structure in which the microparticles 20 are encapsulated in the internal space of the three-dimensional structure formed by the thin film 1. Although the three-dimensional structure 100 is a tubular structure in the present embodiment, the three-dimensional structure of the present invention is not limited to a tubular structure. For example, it can be configured as various three-dimensional structures such as biological tissue-like structures.

As shown in FIGS. 1 and 2, in the present embodiment, the thin film 1 is composed of the first thin film layer 10 and the second thin film layer 11.

The thin film layer 10 and the thin film layer 11 constituting the thin film 1 are composed of a highly biocompatible polymer material selected from polyparaxylene (parylene) and silk fibroin.

Further, for the thin film layer 10 and the thin film layer 11, a polymer material with high transparency may be used. If a polymer material having high transparency is used for the thin film layer 10 and the thin film layer 11, since the optical path is not blocked at the time of observation with a microscope, observation inside the structure becomes possible in any type of microscope regardless of upright type or inverted type. In addition, if a super-resolution microscope is used, it is also possible to observe the behavior of finer cells and the activity of proteins in cells with fluorescence. When the microparticle 20 is adherent cells, it is preferable to use a cell adhesive polymer material for the thin film layer 11.

The thin film layer 10 and the thin film layer 11 have different mechanical strengths from each other. Examples of the mechanical strengths include, for example, elastic modulus. Therefore, it is preferable to form the thin film layer 10 and the thin film layer 11 using polymer materials having swelling ratios different from each other. For example, when the three-dimensional structure 100 is a tubular structure, it is preferable to use a material having a large swelling ratio for the thin film layer 10 and a material having a small swelling ratio for the thin film layer 11. Examples of combinations of such thin film layers include, for example, one in which the thin film layer 10 is composed of a silk fibroin gel and the thin film layer 11 is composed of parylene.

The thickness of the thin film 1 formed by a plurality of thin film layers is not particularly limited, but it is preferable to set the thickness to such a level that the permeability of oxygen or substance to the internal space of the three-dimensional structure is not prevented. For example, the thickness of the thin film 1 can be set preferably from 15 to 400 nm, more preferably from 20 to 300 nm, and still more preferably from 20 to 200 nm. In this case, a cell with a diameter of 10 µm scale can be suitably encapsulated, and bending of the thin film 1 is not prevented. In order to set the thickness of the thin film 1 as described above, for example, the thickness of the thin film layer 10 can be set preferably from 10 to 350 nm, more preferably from 15 to 250 nm, and still more preferably from 50 to 200 nm, and the thickness of the layer 11 can be set preferably from 5 to 200 nm, more preferably from 10 to 150 nm, and still more preferably from 20 to 100 nm.

Further, the surface of the thin film 1 may be formed with an arbitrary two-dimensional plane pattern. For example, an arbitrary two-dimensional shape can be formed by patterning using a lithography technique. The size of the pattern is preferably 50 µm or more. For example, a pattern of an arbitrary two-dimensional shape may be formed on the surface of the thin film 1 depending on the type of cells and the number of cells to be encapsulated. Further, in the case where a cell is encapsulated as the microparticle 20 in the three-dimensional structure 100, depending on the type of the cells, a pattern may be formed on the surface of the thin film 1 so that the shape of the internal space of the three-dimensional structure 100 becomes a biological tissue-like structure. For example, a pattern can be formed on the surface of the thin film 1 so as to configure a shape of an internal space simulating a biological tissue such as a hollow vascular tissue composed of epithelial cells or a fibrous nerve tissue.

The microparticle 20 to be encapsulated in the three-dimensional structure 100 is not particularly limited as long as it is a fine particle having a size of 1 µm or less. Examples of the microparticle 20 include plant and animal cells, bacteria, parasite bodies, microbeads, microbubbles, spherical lipid bilayer membranes (liposomes) and nanoparticles. Among the plant and animal cells, preferable examples include adherent cells, and the like. Examples of the adherent cells include, but are not limited to, nerve cells, cardiac myocytes, and the like.

In the embodiment shown in FIG. 2, the microparticles 20 are adhered to the thin film 1 by a modified protein layer 21. In the case of adhering the microparticle 20 to the surface of the thin film layer 11 as described above, the surface of the thin film layer 11 may be modified with a material having high affinity with the microparticle 20. For example, when the microparticle 20 is an adherent cell, surface modification of the thin film layer 11 can be performed by using an extracellular matrix such as fibronectin, collagen, laminin or the like. By applying such surface modification to the thin film layer 11, it is possible to maintain cell adhesiveness for a longer period of time. The extracellular matrix and the like used for surface modification of the thin film layer 11 are not particularly limited, and a suitable extracellular matrix or the like can be appropriately selected according to the type of the adherent cell. For example, fibronectin or the like can be suitably used in the case of a cultured cell of an established cell line, and laminin or the like can be suitably used in the case of a nerve cell.

The amount of the microparticles 20 to be encapsulated in the three-dimensional structure 100 is not particularly limited, and an appropriate amount can be suitably encapsulated in accordance with the application. When the microparticles 20 are cells, the cells encapsulated in the three-dimensional structure 100 grow according to the shape of the internal space of the three-dimensional structure 100. Therefore, by making the shape of the internal space of the three-dimensional structure 100 as a biological tissue-like structure, the encapsulated cells proliferate to form a biological tissue-like structure. In the three-dimensional structure of the present invention, since the thin film constituting the three-dimensional structure is formed of a polymer material having high biocompatibility, it is possible to culture cells for a long period of time.

Further, when the three-dimensional structure 100 in which cells are encapsulated as the microparticles 20 is moved onto the culture substrate where the cells have been previously cultured, and is cultured, cell processes, axons, cell bodies and the like are extended from the inside of the three-dimensional structure 100 to the outside of the three-dimensional structure 100. Intercellular interactions can occur between the cells encapsulated in the three-dimensional structure 100 and the cells existing outside the three-dimensional structure via structures of these cell processes, axons, cell bodies and the like.

The three-dimensional structure of the present invention is different from the conventional thin film three-dimensional structure from the viewpoints that: (i) the constituting thin film is composed of a highly biocompatible polymer material; (ii) since cells can be cultured on the thin film, the cultured cells can be encapsulated in the three-dimensional thin film structure in a self-assembling manner; and (iii) when encapsulating cells, the encapsulated cells can function as a biological tissue.

As described above, in the three-dimensional structure of the present invention, when cells are encapsulated, the encapsulated cells can function as a biological tissue, and a biological tissue-like structure can be formed by the encapsulated cells. Cells inside the three-dimensional structure can also interact with cells outside the three-dimensional structure. For this reason, the three-dimensional structure encapsulating cells can be applied, as a biological tissue-like structure, to transplanted tissues (grafts) for repairing nerve tissues such as epilepsy and spinal cord injuries, transplanted tissues (grafts) for repairing myocardial tissues damaged by myocardial infarction, and the like. Moreover, it can be applied to drug screening or the like as a pseudo biological tissue to test the drug response. In addition, by designing a three-dimensional structure having a bent hinge structure, it is also possible to obtain a three-dimensional structure that realizes capture of a target cell, adsorption to a tissue surface of a target cell, an actuator function for holding a target cell, or the like. Furthermore, the three-dimensional structure of the present invention can also be applied as an element for an in vivo implantable device.

### <Method for producing three-dimensional structure>

The three-dimensional structure of the present invention is composed of a polymer materials. Due to its low rigidity, although it is possible to form a thin film, it is difficult for the polymer material to process the formed thin film or to form an intensity distribution. Therefore, there are still few reports on techniques for fabricating three-dimensional shapes using polymer thin films.

Accordingly, in the present invention, a phenomenon in which the polymer thin film is assembled into a three-dimensional shape in a self-assembling manner, by forming a polymer thin film composed of a plurality of layers using a lithographic technique or the like and creating a structure that generates a stress distribution in the thickness direction inside the polymer thin film, is utilized. That is, one embodiment of the present invention is a method for producing a three-dimensional structure encapsulating a microparticle, the method including: a step of forming a polymer film having a plurality of layers; a step of floating the microparticle over a surface of the aforementioned polymer film having the plurality of layers; and a step of generating a stress distribution in the thickness direction in the aforementioned polymer film having the plurality of layers to form a three-dimensional structure in a self-assembling manner in the aforementioned polymer film having the plurality of layers. Hereinafter, the method for producing a three-dimensional structure of the present invention will be described with reference to drawings showing a preferred embodiment of the present invention.

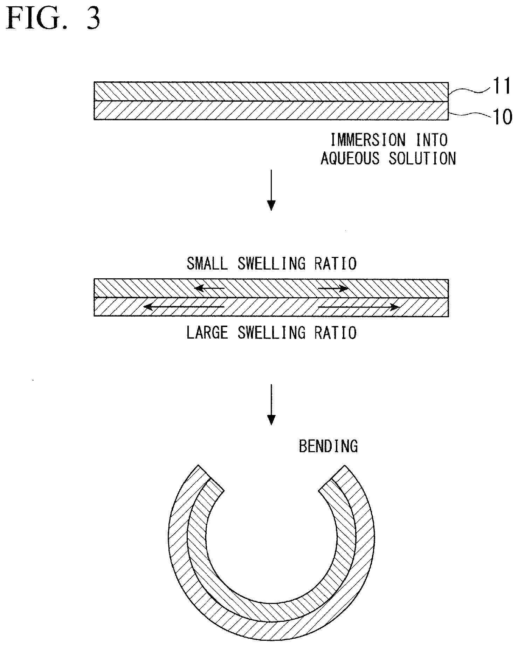

FIG. 3 is a conceptual diagram of bending by a laminated structure of two layers of polymer thin films with different swelling ratios. The reference numerals in the drawing are the same as those in FIG. 1 and FIG. 2. First, assembly of a three-dimensional shape in a self-assembling manner by a polymer thin film having a plurality of layers in which the swelling ratios of the respective layers are different will be described with reference to FIG. 3.

In the structure illustrated in FIG. 3, the thin film layer 10 and the thin film layer 11 are formed of polymer materials having swelling ratios different from each other. The swelling ratio of the thin film layer 10 is larger than the swelling ratio of the thin film layer 11. Therefore, when the thin film 1 composed of the thin film layer 10 and the thin film layer 11 is immersed in an aqueous solution, each layer swells by absorbing water, but the amount of change in volume due to swelling is larger in the thin film layer 10 than in the thin film layer 11. Using the difference in the amount of change in volume as a driving force, the thin film 1 is bent in such a manner that the thin film layer 11 becomes an inner layer and the thin film layer 10 becomes an outer layer to form a three-dimensional structure.

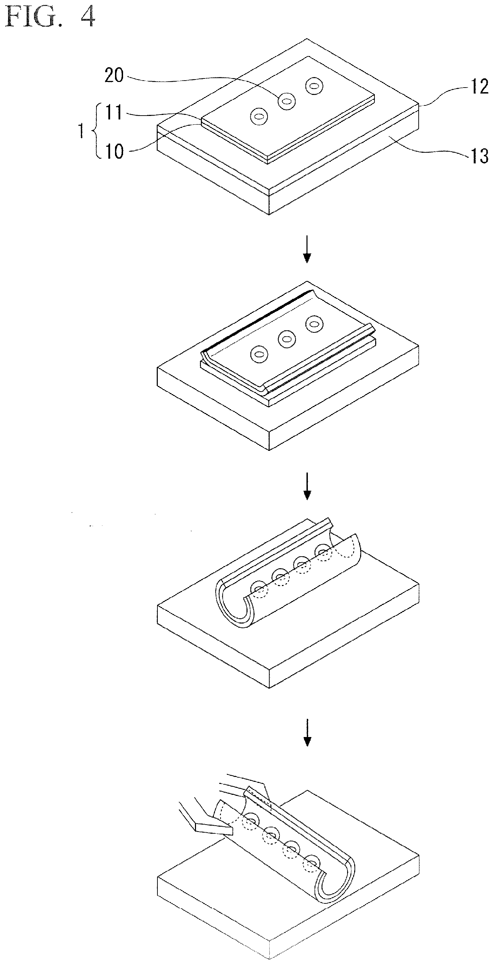

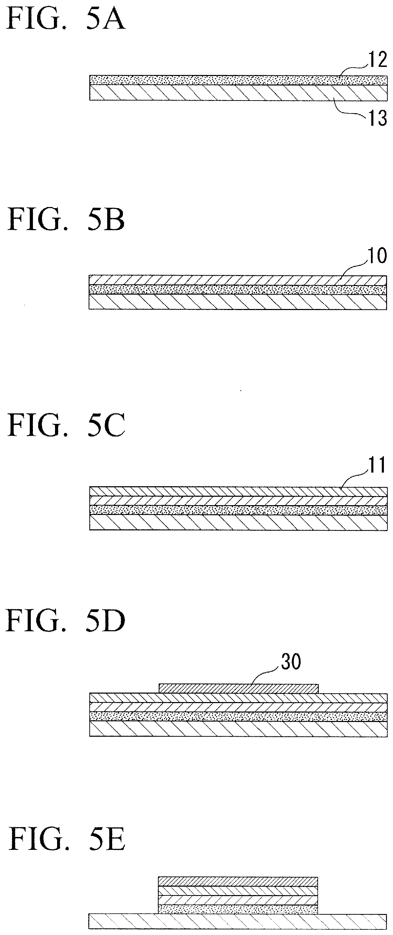

Next, one embodiment of the method for producing a three-dimensional structure of the present invention will be described with reference to FIG. 4 and FIGS. 5A to 5J. FIG. 4 is a conceptual diagram showing one embodiment of self-assembly into a three-dimensional shape using a thin film having a two-layer structure and encapsulation of microparticles. Further, FIGS. 5A to 5J are an example of a process diagram of formation of a three-dimensional structure encapsulating microparticles. In the drawings, reference numerals 12, 13 and 30 denote a sacrificial layer, a substrate and a photoresist film, respectively. Other reference numerals are the same as those in FIGS. 1 and 2.

In the embodiment shown in FIG. 4 and FIGS. 5A to 5J, the sacrificial layer 12 formed between the thin film 1 and the substrate 13 is used in order to release the thin film 1 from the substrate 13 to form a three-dimensional structure. Therefore, first, as shown in FIG. 5A, the sacrificial layer 12 is formed on the substrate 13. The method of forming the sacrificial layer 12 is not particularly limited, and spin coating, chemical vapor deposition (CVD), inkjet printing, a vapor deposition method, an electrospray method, or the like can be used.

The material of the substrate 13 is not particularly limited, but it is preferable to use a material having high surface flatness. Further, when observing the three-dimensional structure 100 encapsulating a cell on the substrate 13 with a fluorescence microscope, it is preferable to use a material which does not hinder the fluorescence intensity of the cell by the fluorescence microscope. Moreover, it is preferable that the wavelength absorption bands in a spectrophotometer and an infrared spectrometer do not overlap with those of the thin film layer 10. Examples of such materials include, for example, silicon, soda glass, quartz, magnesium oxide and sapphire. It should be noted that in the examples of FIGS. 5A to 5J, a glass substrate is used as the substrate 13.

The thickness of the substrate 13 is not particularly limited, and can be set to, for example, 50 to 200 µm. Further, the surface of the substrate 13 may be modified with PEG, 2-methacryloyloxyethyl phosphorylcholine (MPC) polymer or the like for the purpose of suppressing nonspecific adsorption of a cell.

The material of the sacrificial layer 12 is not particularly limited, but it is preferable to use a physical gel capable of undergoing sol-gel transition. It is also preferable that the solution or the stimulus such as light used for sol-gel transition does not exhibit cytotoxicity. Examples of such gels include gels decomposed by changes in light, heat, pH and the like. Specific examples thereof include poly(N-isopropylacrylamide) (PNIPAM), azobenzene-modified polymer gels, and the like. In addition, gels which are decomposed by the action of chelating agents, enzymes or the like can also be used. As such a gel, for example, a calcium alginate gel and the like can be mentioned. It should be noted that in the examples shown in FIGS. 5A to 5J, a calcium alginate gel is used as the sacrificial layer 12. The thickness of the sacrificial layer 12 is not particularly limited, and can be set to, for example, 20 to 200 nm.

Next, as shown in FIG. 5B, the thin film layer 10 is formed on the sacrificial layer 12. The method of forming the thin film layer 10 is not particularly limited, and spin coating, CVD, inkjet printing, a vapor deposition method, an electrospray method, or the like can be used. The material and the thickness of the thin film layer 10 may be set as described above. As the material of the thin film layer 10, for example, a polymer material which swells when immersed in a solution and induces a change in volume is preferable. It should be noted that in the examples of FIGS. 5A to 5J, a silk fibroin gel is used as the thin film layer 10.

Next, as shown in FIG. 5C, the thin film layer 11 is formed on the thin film layer 10. The method of forming the thin film layer 11 is not particularly limited, and CVD, spin coating, inkjet printing, a vapor deposition method, an electrospray method, or the like can be used. The material and the thickness of the thin film layer 11 may be set as described above. As the material of the thin film layer 11, for example, a polymer material which is not induced to have a large volume change as compared with the thin film layer 10 when immersed in a solution is preferable. Alternatively, a polymer material in which a volume change opposite to that of the thin film layer 10 is induced is preferable. It should be noted that in the examples of FIGS. 5A to 5J, parylene is used as the thin film layer 11.

By using polymer materials having different swelling ratios for the thin film layer 10 and the thin film layer 11 as described above, when immersed in a solution, a difference in the volume change due to swelling occurs between the thin film layer 10 and the thin film layer 11, and a stress distribution is generated in the thickness direction. This stress distribution serves as a driving force, and when the sacrificial layer 12 is decomposed and the thin film 1 is released from the substrate 13 in a later step, the thin film 1 forms a three-dimensional shape in a self-assembling manner.

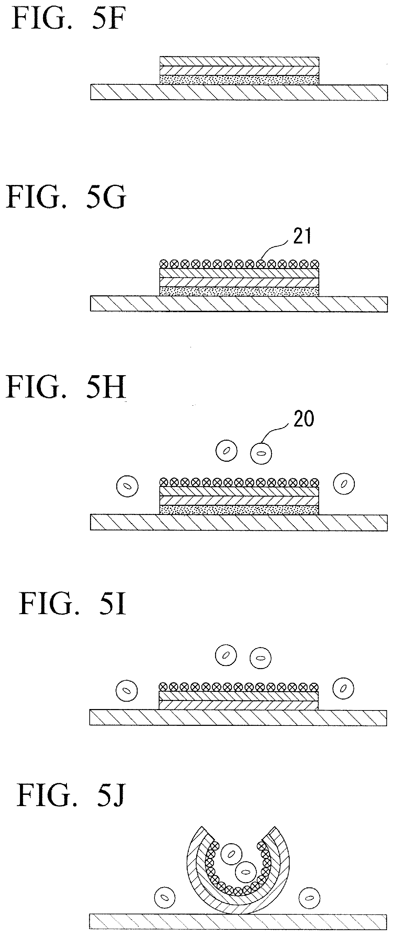

Next, as shown in FIGS. 5D to 5F, a pattern is formed on the thin film 1 as necessary. As a pattern forming method, for example, a microfabrication technique such as a photolithography method, an electron beam lithography method, a dry etching method, or the like can be applied. In the example of FIG. 5D, a photoresist film 30 is formed on the thin film layer 11, and ultraviolet rays are irradiated through a photomask of an arbitrary shape, and a physical mask is patterned. After that, etching may be performed as shown in FIG. 5E, and the photomask is removed as shown in FIG. 5F. It should be noted that the etching may be performed until reaching the substrate 13 or may be performed until reaching the sacrificial layer 12. Although the pattern formation on the thin film 1 is optional, by designing a two-dimensional planar pattern on the thin film 1, it is possible to freely change the three-dimensional shape assembled in a self-assembling manner. For example, by forming a pattern so that the internal space of the three-dimensional structure after assembly becomes a biological tissue-like structure, when cells are encapsulated in the three-dimensional structure, it becomes possible to grow the cells along the biological tissue-like structure. For example, a design corresponding to the shape of an actual biological tissue such as a hollow vascular tissue composed of epithelial cells, a fibrous nerve tissue composed of neuronal cells and a heart-shaped myocardial tissue composed of cardiac myocytes becomes possible.

Next, as shown in FIG. 5G, the surface of the thin film layer 11 may be modified with a material having high affinity with the microparticle 20, if necessary. In the example of FIG. 5G, the modified protein layer 21 is formed on the surface of the thin film layer 11. Materials and the like used for modification may be as described above. It should be noted that in the example of FIG. 5G, modification is performed with fibronectin or laminin.

Next, as shown in FIG. 5H, a suspension of the microparticle 20 is added onto the thin film layer 11, and the microparticle 20 is floated over the thin film layer 11. At this time, by adjusting the concentration of the microparticles 20 in the suspension, the number of microparticles 20 encapsulated in the three-dimensional structure after the assembly can be controlled.

Next, as shown in FIG. 5I, the sacrificial layer 12 is decomposed. Depending on the material of the sacrificial layer 12, an appropriate method may be adopted for decomposition of the sacrificial layer 12. For example, if the sacrificial layer 12 is a gel which is decomposed by changing light, heat or pH, the sacrificial layer 12 can be decomposed by changing light, heat or pH. In addition, if the sacrificial layer 12 is a gel which is decomposed by the action of a chelating agent or an enzyme, the sacrificial layer 12 can be decomposed by the action of a chelating agent or an enzyme. It should be noted that in the example of FIG. 5I in which the sacrificial layer 12 is composed of a calcium alginate gel, the sacrificial layer 12 can be decomposed by adding a chelating agent such as sodium citrate or EDTA, an enzyme called arginase that specifically degrades a calcium alginate gel, or the like.

By decomposing the sacrificial layer 12 as described above, the thin film 1 is released from the substrate 13 as shown in FIG. 5J, and forms a three-dimensional structure of an arbitrary shape. At that time, the microparticle 20 that have been present on the thin film 1 is encapsulated in the internal space of the three-dimensional structure.

By decomposing the sacrificial layer 12 by a stimulus that has no cytotoxicity, even when a cell is used as the microparticle 20, it becomes possible to add the cell onto the thin film 1 immediately before decomposition operation of the sacrificial layer 12. At this time, by changing the cell concentration of the cell suspension on the thin film 1, it is possible to control the number of cells encapsulated in the three-dimensional structure. Further, since the cells are encapsulated in the three-dimensional structure simultaneously with the assembly of the three-dimensional structure, a large number of cells can be collectively encapsulated in the three-dimensional structure. Therefore, as compared with the conventional method that relies on accidental entry of cells into the three-dimensional structure, the introduction efficiency of cells into the three-dimensional structure can be remarkably improved.

Although the embodiments of the present invention have been described above in detail with reference to the drawings, the specific configuration is not limited to these embodiments, and other designs and the like are also included insofar as they do not depart from which is defined by the claims.

### Examples

Hereinafter, the present invention will be described in more detail with reference to specific examples. However, the present invention is not limited in any way by the following examples.

### [Example 1] Preparation of thin film capable of self-assembling into three-dimensional structure

Fabrication of a thin film capable of self-assembling into a three-dimensional structure was carried out according to the process shown in FIGS. 5A to 5F. In this example, a glass substrate was used as a substrate 13, and a calcium alginate gel was used as a sacrificial layer 12. First, a sodium alginate solution was spin-coated on the substrate 13 which was a glass substrate. Then, the spin-coated substrate 13 was immersed in a 100 mM calcium chloride solution to thereby form a sacrificial layer 12 composed of a physical gel of calcium alginate (FIG. 5A). The thickness of the calcium alginate gel can be controlled by changing the concentration of the sodium alginate solution and the spin coating rate, and in the present example, a gel layer having a thickness of 40 nm was formed by spin-coating a 2 wt% sodium alginate solution at 3,000 rpm.

Next, a thin film layer 10 was formed on the sacrificial layer 12. As a gel constituting the thin film layer 10, a silk fibroin gel was used. Silk fibroin was dissolved in water for use and filtered to remove molecules larger than 200 nm. The silk fibroin solution prepared as described above was spin-coated on the surface of the sacrificial layer 12, followed by immersion into a methanol solution to thereby form a thin film layer 10 composed of a silk fibroin gel (FIG. 5B). The thickness of the silk fibroin gel can be controlled by changing the concentration of the silk fibroin solution and the spin coating rate, and in the present example, a gel layer having a thickness of about 200 nm was formed by spin-coating a 40 mg/mL silk fibroin solution at 1,000 rpm.

Next, a thin film layer 11 was formed on the thin film layer 10. On the surface of the thin film layer 10, a dimer of paraxylene was grown by CVD to thereby form a thin film layer 11 composed of a parylene thin film (FIG. 5C). The thickness of the thin film layer 11 can be controlled by the input weight of the paraxylene dimer, and in the present example, a parylene layer of about 50 nm was formed by growing 50 mg of paraxylene dimer on the thin film layer 10 by CVD.

Next, a positive type photoresist (S1813) was spin-coated on the thin film layer 11 and irradiated with ultraviolet light through a photomask, thereby patterning a physical mask on the thin film layer 11 (FIG. 5D). Then, etching was carried out in an asher with oxygen plasma (FIG. 5E). The etching was performed until reaching the substrate 13. Finally, the photomask was removed with acetone to expose the thin film layer 11 which was a parylene layer (FIG. 5F).

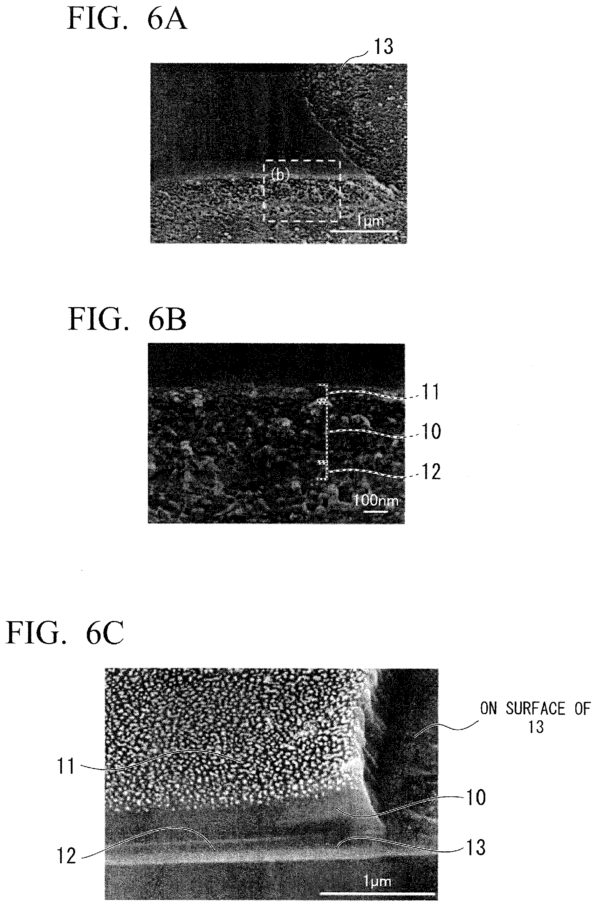

FIGS. 6A and 6B show electron microscope (SEM) images of the thin film pattern formed as described above. FIG. 6B is an enlarged image of a region surrounded by the dotted line in FIG. 6A. From the SEM images in FIGS. 6A and 6B, it was confirmed that the respective layers of the thin film layer 10, the thin film layer 11 and the sacrificial layer 12 were laminated in a planar manner. In addition, although each layer was cut by the etching operation, it was confirmed that fine particles were present on the substrate 13. It is considered that these were remnant of the calcium alginate gel which could not be removed even by the etching operation.

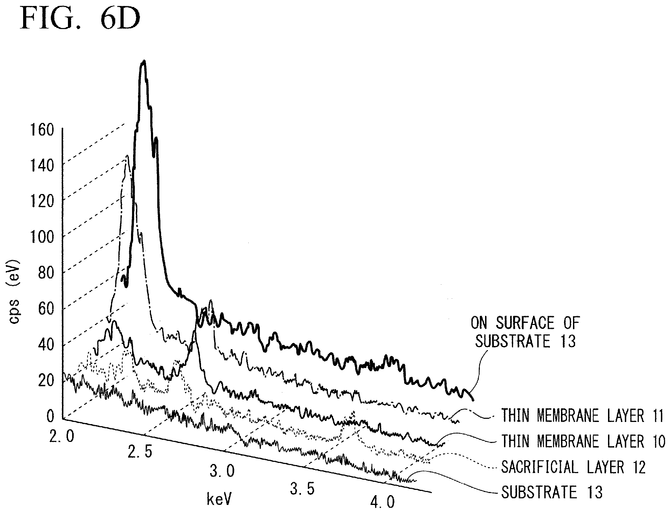

FIG. 6C shows a SEM image of the cross section after cutting the thin film layer by a focused ion beam (FIB). Further, in the cross section, confirmation of the localization and identification of specific elements constituting these thin film layers, the substrate 13, and the microparticles on the substrate 13 were carried out by energy dispersive X-ray analysis (EDX) (FIG. 6D). As a result, chlorine (CI) specific to parylene was observed in the thin film layer 11, calcium (Ca) specific to the calcium alginate gel was observed in the sacrificial layer 12, and silicon (Si) was observed in the substrate 13, respectively. In addition, the presence of gold (Au) sputtered for the SEM observation was confirmed in the thin film layer 11 and the substrate 13 after etching, and the presence of calcium (Ca) specific to the calcium alginate gel was observed in the microparticles on the substrate 13 after etching.

### [Example 2] Self-assembly of three-dimensional structure by thin film

Self-assembly of a three-dimensional structure encapsulating cells was performed according to the process shown in FIGS. 5G to 5J. The substrate 13, prepared in Example 1, to which the thin film 1 and the sacrificial layer 12 were adhered was immersed in a protein solution to subject the surface of the parylene film of the thin film layer 11 to protein modification (FIG. 5G). The type of protein modification is appropriately selected depending on the type of cells to be encapsulated. In the present example, in order to induce adhesion of cultured cells of an established cell line, modification of the thin film layer 11 was carried out using a 1 mg/mL fibronectin solution. The 1 mg/mL fibronectin solution was simultaneously added into the culture medium at the time of seeding the cultured cells of an established cell line so that the final concentration was adjusted to 1 µg/mL. In addition, in order to induce adhesion of primary neurons, the thin film layer 11 was modified using a 1 mg/mL laminin solution. The 1 mg/mL laminin solution was simultaneously added into the culture medium at the time of seeding the primary neurons so that the final concentration was adjusted to 1 mg/mL. The cell culture medium prepared as described above was seeded on the thin film 1, and the cells were floated over the surface of the thin film layer 11 (FIG. 5H). It should be noted that it is possible to control the number of cells to be encapsulated by changing the number of cells to be seeded before self-assembling the thin film 1.

Next, a chelating agent was added to dissolve a calcium alginate gel layer of the sacrificial layer 12 (FIG. 5I). Although the chelating agent should not be cytotoxic, in the present example, an EDTA solution was used as a chelating agent. A 0.05 mol/mL EDTA solution was added to a final concentration of 0.001 mol/L to dissolve the sacrificial layer 12, and the thin film 1 was liberated from the substrate 13.

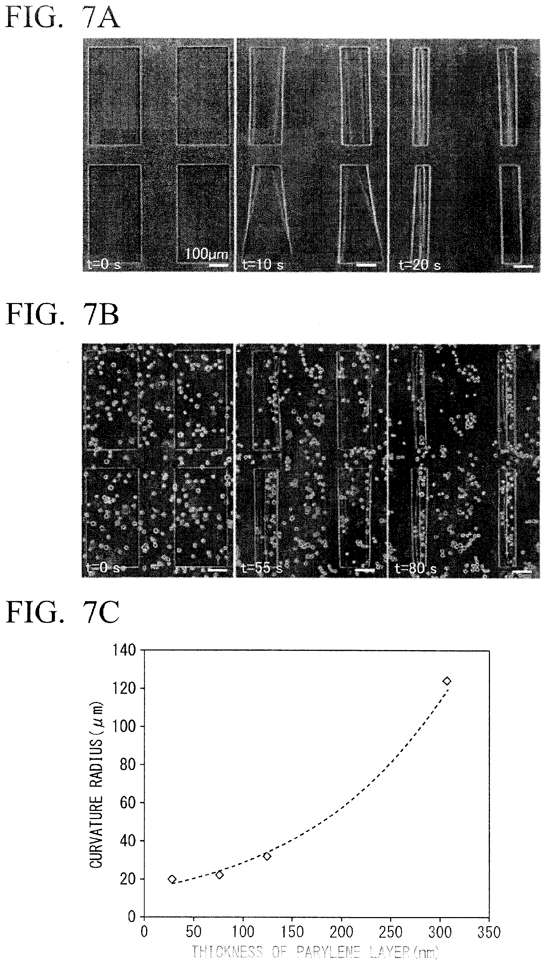

When the sacrificial layer 12 was dissolved by the addition of the EDTA solution, the thin film 1 was released from the substrate 13, and self-assembly into a tubular structure occurred (FIG. 5J). FIGS. 7A and 7B are phase contrast microscope images showing a state of self-assembly of the thin film 1. Observations were made in the absence of cells (FIG. 7A) and in the presence of cells (FIG. 7B), but in either case, the thin film 1 was gradually detached from the substrate 13 after the addition of the EDTA solution, and it was observed that the reaction proceeded gradually to the central part. At this time, since the reaction proceeds isotropically, it was observed that the reaction in the minor axis direction was completed more quickly than that in the major axis direction, and the bending of the thin film in the minor axis direction was induced. As a result, a tubular structure was obtained in a state in which the length in the major axis direction was maintained.

The time from the addition of the EDTA solution to the completion of the tubular structure can be controlled by the final concentration of the EDTA solution to be added and the type of the solution in which the substrate was immersed. In the present example, by immersing the sacrificial layer 12 composed of a calcium alginate gel having a length of 200 µm, a width of 400 µm and a thickness of 40 nm in 200 µL of pure water and adding a 0.5 M EDTA solution, it was possible to remove the sacrificial layer 12 within about 20 seconds (FIG. 7A). In addition, along with the bending of the thin film 1, the cells floated over the thin film 1 were incorporated into the internal space of the tubular structure (FIG. 7B). It was confirmed that the cells encapsulated in the tubular structure did not change the position in the internal space of the tubular structure even if subsequent operations such as a solution exchange operation and handling of the structure were performed.

Since the bending phenomenon of the thin film 1 is caused by the stress distribution in the thickness direction of the thin film 1, by changing the volumes of the thin film layer 10 and the thin film layer 11 constituting the thin film 1, the curvature at the time of bending the thin film 1 can be controlled. FIG. 7C shows a correlation between the curvature radius of the thin film 1 and the thickness of the thin film layer 11 composed of a parylene layer. It was observed that as the thickness of the parylene layer of the thin film layer 11 increased, the second moment of area of the structure increased under certain stress, making it more difficult to bend.

The tubular structure produced as described above is completely separated from the substrate 13. This enables handling such as collection and transfer by pipetting. Furthermore, it is also possible to bring a plurality of tubular structures close to each other by using a glass capillary. Therefore, a tubular structure encapsulating cells is used as a graft, and it can be applied to transportation or transplantation to a target living tissue or the like.

### [Example 3] Culture of adherent cell encapsulated in tubular structure

In the present example, Chinese hamster ovary-derived (CHO) cells and human embryonic kidney-derived (HEK) cells, which were cultured cells of established cell lines, were used as cells to be encapsulated in the tubular structure. Both cells were cultured using a Dulbecco's modified Eagle medium (DMEM) containing 10% fetal bovine serum (FBS) as a culture medium. Both cells were cultured in a humid environment in which the temperature was kept at 37°C and the carbon dioxide concentration was maintained at 5%.

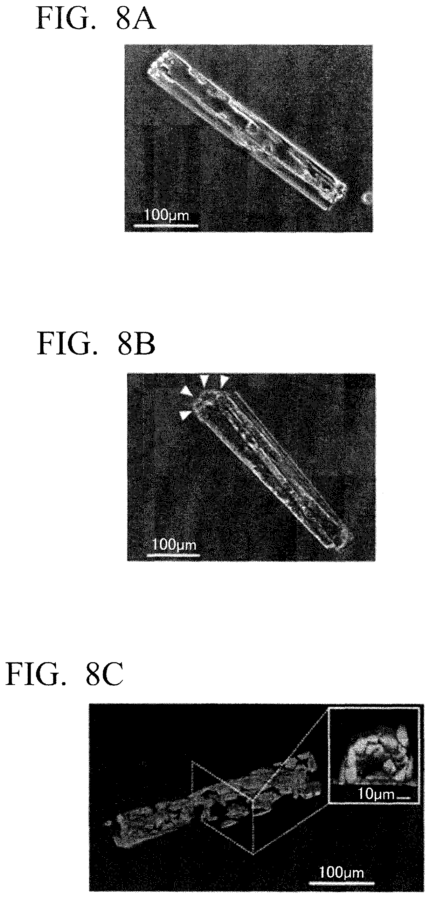

Preparation of the tubular structure and encapsulation of the cells were carried out as in Example 1 and Example 2. After one week from encapsulation into the tubular structure, viability of the cells were evaluated, and survival of both CHO cells and HEK cells in the tubular structure was confirmed. Further, in the CHO cells and the HEK cells which are cultured cells of established cell lines that grow repeatedly and endlessly, it was observed that the inside of the space of the tubular structure was filled with cells along with the cell proliferation, thereby forming cell aggregates. In addition, depending on the type of cells, the structure of the formed cell aggregate was different. In the CHO cells, the cells adhered only to the surface of the thin film layer 11 and showed a biological tissue-like structure with a hollow structure (FIG. 8A). On the other hand, in the HEK cells in which the cells are strongly adhered to each other, adhesion of the cells to each other is stronger than adhesion to the surface of the thin film layer 11, and cell aggregates (spheroids) in which the cells were aggregated were formed (FIG. 8B) while maintaining the structure of the tubular structure. It was observed that the cell aggregates formed in the HEK cells increased in volume with the culture, and the proliferation proceeded to the outside of the tubular structure and extended to the substrate 13 (arrow in FIG. 8B). In addition, FIG. 8C shows a confocal microscope image of a tubular structure encapsulating CHO cells. In this image, the cells are fluorescently labeled with Calcein-AM, and the entire cytoplasm is stained. Based on this image, it was observed that the cell body was adhered to the thin film wall surface and was localized on the wall surface.

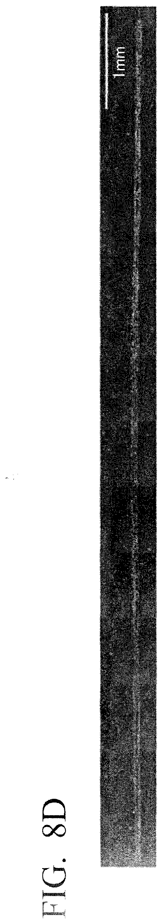

In addition, based on the tubular structure of the present example, it is also possible to produce a longer biological tissue-like structure by making the major axis direction longer. The tubular structure shown in FIG. 8D is an example in which a large biological tissue-like structure of 1 cm or more is produced. In recent years, although many researches have been conducted to prepare cell aggregates and try to apply them to regenerative medicine, it is reported that when the size of the aggregates of cells reaches 200 µm or more, the permeability of oxygen and nutrients decreases, and the cell death is induced from the inside of the aggregates. In the three-dimensional structure of the present invention, since the thickness of the thin film 1 can be controlled, the permeability of oxygen and nutrients can be appropriately maintained. In addition, since it is possible to control the diameter without disorderly enlarging the structure of the biological tissue-like structure, long-term culture of cells inside the three-dimensional structure became possible.

In the present example, cell bodies were encapsulated inside the tubular structure during the culture period, and it was possible to manipulate while maintaining that state. In addition, in the thin film 1 encapsulating the cells, the three-dimensional shape did not collapse even when the culture was continued at 37°C in the DMEM medium. With the use of a glass capillary, cell aggregates encapsulated in the three-dimensional structure can be moved to the x-y plane without changing the three-dimensional structure, and transplantation to places where different cell groups were present was also possible while the cells were encapsulated in the three-dimensional structure. Furthermore, it was confirmed that the cell aggregates could be rotated in the minor axis direction while being encapsulated in the tubular structure, the angle (inclination) on the z axis could be controlled, and it can also be applied to multi-angle observation of cells.

### [Example 4] Culture of primary neurons encapsulated in tubular structure

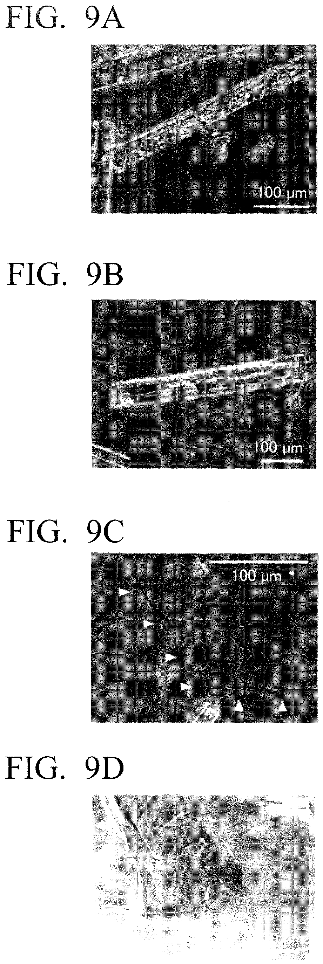

In the present example, hippocampal cells and cerebral cortical cell which were primary neurons isolated from a rat brain tissue were used. As shown in FIG. 9A, when a large number of cells were encapsulated in a single tubular structure, association of cells with each other was started along with the culture, and cell aggregates were formed. In the case of primary neurons, although adhesion of cells to each other is induced along with the long-term culture, adhesion to the inner surface of the tubular structure is also maintained at the same time, and while this state is maintained, elongation of neurites or axons only in the internal space of the tubular structure was observed (FIG. 9B). In both hippocampal cells and cerebral cortical cells, it was confirmed that during the culture period of one month or more, stable cell body morphology and an axon extension state were maintained inside the tubular structure, and the cell death was not induced inside the tubular structure.

Since primary cerebral cortical cells and hippocampal cells have slow cell growth rates, the cells could be cultured for a longer period of time of 1 month or more, as compared with the cultured cells of established cell lines, without the cells being protruded from the tubular structure. In addition, since primary neurons extend nerve axons for neurotransmission, it was also confirmed that the cells form cell aggregates inside the tubular structure and then extend the nerve axons to the outside of the tubular structure. In the present example, since the three-dimensional structure was cylindrical and only the two end points thereof were open to the culture medium space, the nerve axons were extended from the end points to the outside of the tubular structure. This indicates that the three-dimensional structure of this example encapsulating the primary neurons not only enables assembly of a nerve tissue-like microstructure but also enables application as an electrical wiring element to transmit electrical signals of the cells unidirectionally in the major axis direction.

It was possible to move the nerve tissue-like cell aggregate of the present example without disrupting the tissue by handling the tubular structure. It was confirmed from the phase contrast microscope image (FIG. 9C) and the SEM image of the freeze-dried sample (FIG. 9D) that the axon extended from the tubular structure onto the surface of the substrate to which the tubular structure was moved. Furthermore, by moving the tubular structure of the present example onto a culture substrate on which different types of cells had previously been cultured, it was confirmed that axons extended from the tubular structure onto the substrate surface in the same manner as described above, and intercellular interactions occurred by binding with the cell bodies that had been present previously on the substrate.

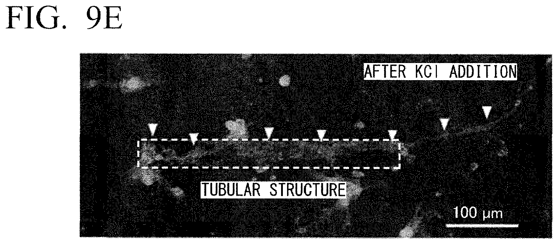

In the primary neurons, there is a difference in ion concentration between the inside and the outside of the cell membrane, and the inside of the membrane is negatively polarized in a stationary state. Since cells have a function of regulating the opening and closing of ion conduction pores according to changes in biomembrane potential, by inducing cell depolarization using a potassium chloride (KCI) solution, it is possible to forcibly activate the voltage-dependent calcium ion channel and allow calcium ions to flow into the cell. Accordingly, after encapsulating and incubating the primary neurons in the tubular structure, a KCI solution was added to induce depolarization. As a result, it was demonstrated that not only nerve cells existing outside the tubular structure but also cells encapsulated in the tubular structure can be stimulated. Furthermore, calcium was labeled with a calcium fluorescent probe Fluo-4, and the permeation of calcium ions in the extracellular fluid into the cell was observed with a confocal microscope. As a result of adding a KCI solution and stimulating the cells, as shown in FIG. 9E, a change in fluorescence intensity was observed not only in the cells outside the tubular structure but also in the cells encapsulated in the tubular structure. In addition, a change in fluorescence intensity was also observed on the junction between the cell encapsulated in the tubular structure and the external cells and on the axon. Furthermore, it was confirmed by the confocal microscope that these changes in fluorescence intensity were synchronized. Further, it was observed that ignition of cells encapsulated in the tubular structure was induced synchronously even after stimulation with the KCI solution, and sustained adhesion between the cells within the minute nerve-like tissue formed inside the tubular structure and the intercellular exchange of electrical signals were confirmed.

### [Example 5] Culture of primary cardiac myocytes encapsulated in tubular structure

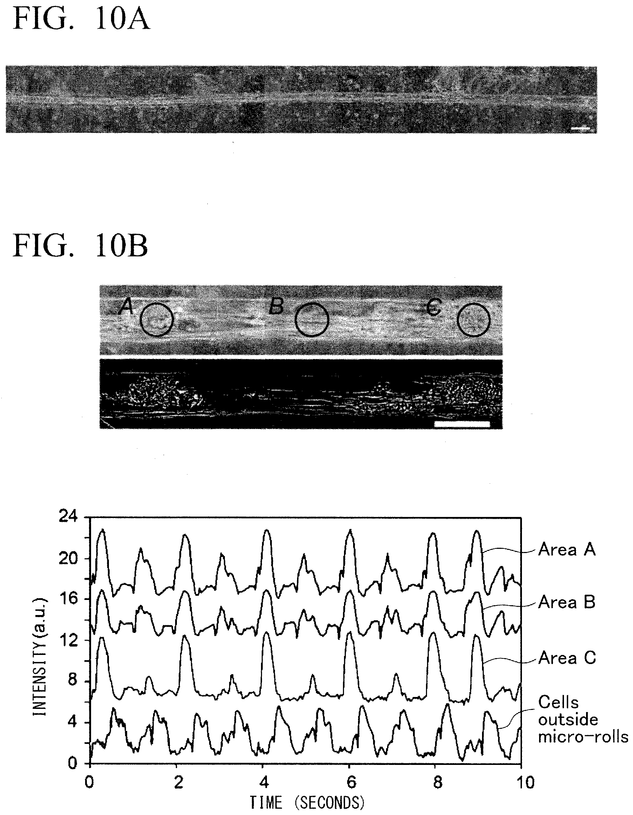

In the present example, primary cardiac myocytes isolated from a rat cardiac tissue were used. As shown in FIG. 10A, when the cardiac myocytes were encapsulated in a single tubular structure, association of cells with each other was started along with the culture as in the case of the primary neurons, and cell aggregates were formed. The cell aggregates were formed in one direction in a fibrous form, and its direction was the same direction as that of the tubular structure. In the case of primary cardiac myocytes, although adhesion of cells to each other is induced along with the long-term culture, adhesion to the inner surface of the tubular structure is also maintained at the same time, and while this state is maintained, formation of cell aggregates only in the internal space of the tubular structure was observed.

In the cardiac myocytes, it was confirmed that during the culture period of one month or more, stable cell aggregate morphology was maintained inside the tubular structure, and the cell death was not induced inside the tubular structure. As shown in FIG. 10B, in the encapsulated primary cardiac myocytes, it was confirmed that the cell aggregates began to beat and that the beat synchronized in cells at any location inside the tubular structure. As a result, it was confirmed that a minute cardiac tissue was successfully reconstructed.

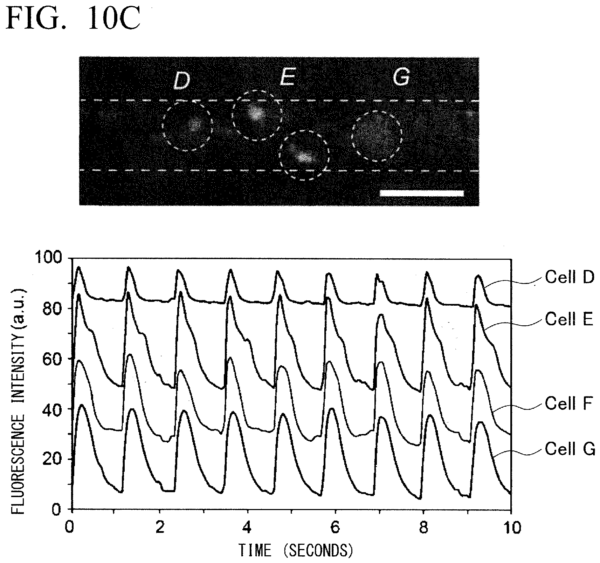

As in the case of primary neurons, in the cardiac myocytes, there is a difference in ion concentration between the inside and the outside of the cell membrane, and the inside of the membrane is negatively polarized in a stationary state. Cells have a function of regulating the opening and closing of ion conduction pores according to changes in biomembrane potential. It is known that the beating of myocardial tissue causes calcium ions to flow into the cell when the cell receives an electrical signal. Accordingly, calcium was labeled with a calcium fluorescent probe Fluo-4, and the permeation of calcium ions in the myocardial extracellular fluid into the cardiac myocytes was observed with a fluorescence microscope. As shown in FIG. 10C, it was observed that changes in fluorescence intensity were also synchronized in cells at any location inside the tubular structure in a manner to be synchronized with the beating. In addition, it was observed that changes in fluorescence intensity were also synchronized only inside the tubular structure.

### [Example 6] Production of three-dimensional structure of various shapes

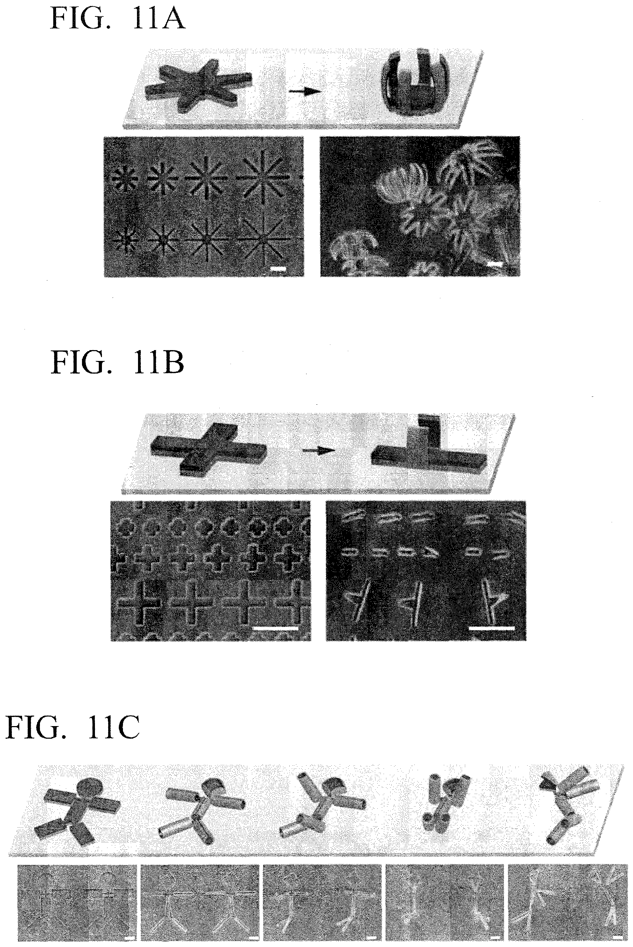

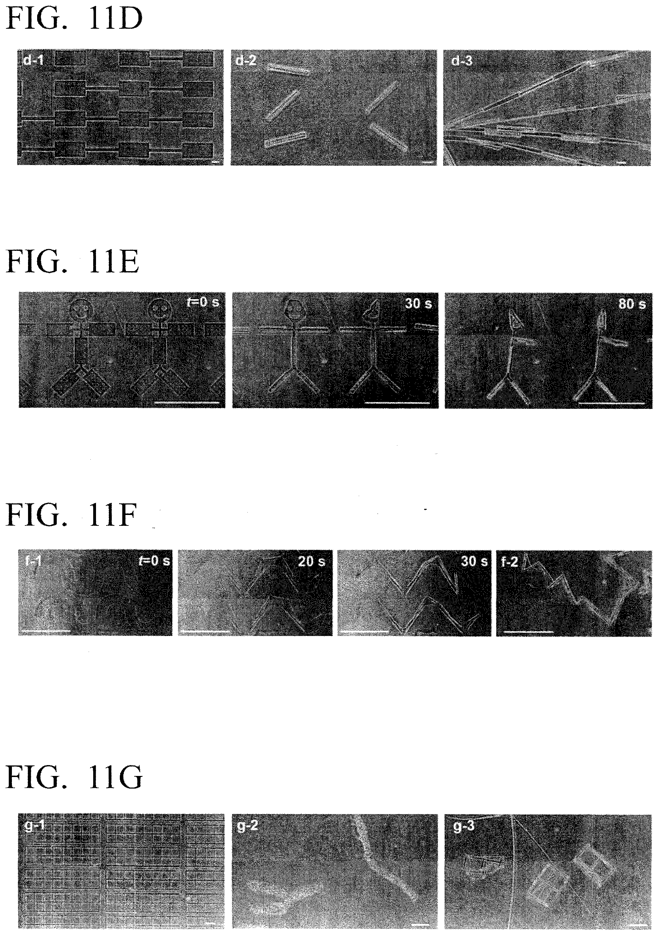

It was confirmed that not only rectangular thin films are self-assembled into tubular structures but also various three dimensional structures can be produced by arbitrarily determining the two-dimensional shape of thin films. FIGS. 11A to 11G show three-dimensional structures self-assembled from thin films of various two-dimensional shapes. A thin film having a radial floral pattern shape formed a three-dimensional structure having a spherical holding gripper structure (FIG. 11A). In the thin film having a cross shape, only one direction of the cross shape was bent to form a three-dimensional structure having a T-shaped structure (FIG. 11B). Furthermore, by joining a cross-shaped thin film to a rectangular thin film, a three-dimensional human-type structure was formed via an unbending joint portion simulating a human form (FIG. 11C). In addition, it was observed that even if pores were formed inside the thin film, it had the same three-dimensional structure as that of the thin film in which pores were not formed (FIGS. 11D and 11E). Therefore, by forming pores in the thin film, a three-dimensional structure which induces the supply of substances from the outside can also be produced. Furthermore, the thin film having a wave-like shape formed a three-dimensional structure having a helical structure (FIG. 11F). In addition, the thin film having a lattice shape formed a three-dimensional structure having a mesh-like net structure (FIG. 11G). From these results, it was shown that by controlling the shape of the thin film, it is possible to produce a three-dimensional structure having various structures.

### [Example 7] Control of curvature radius of tubular structure

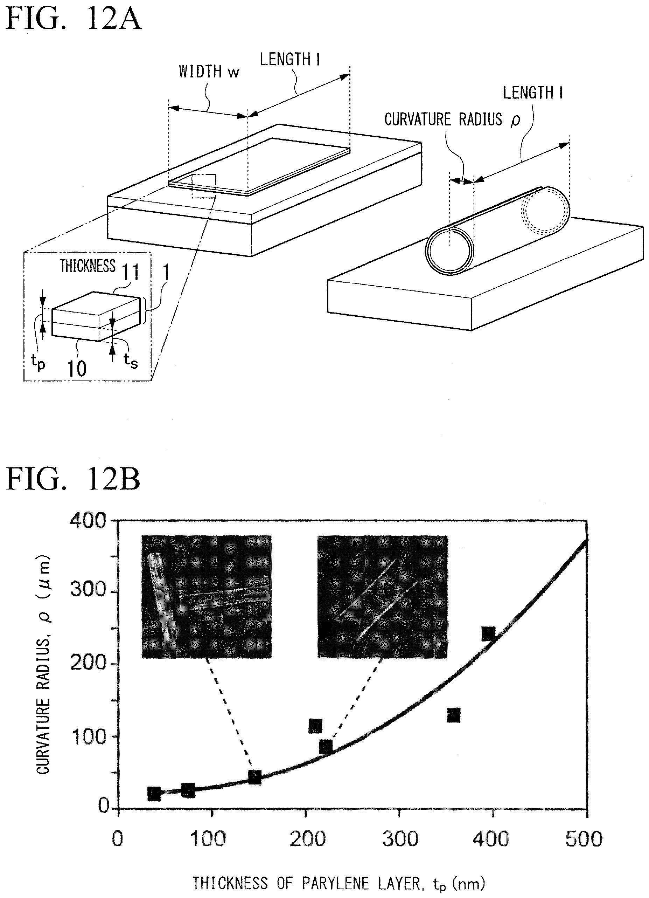

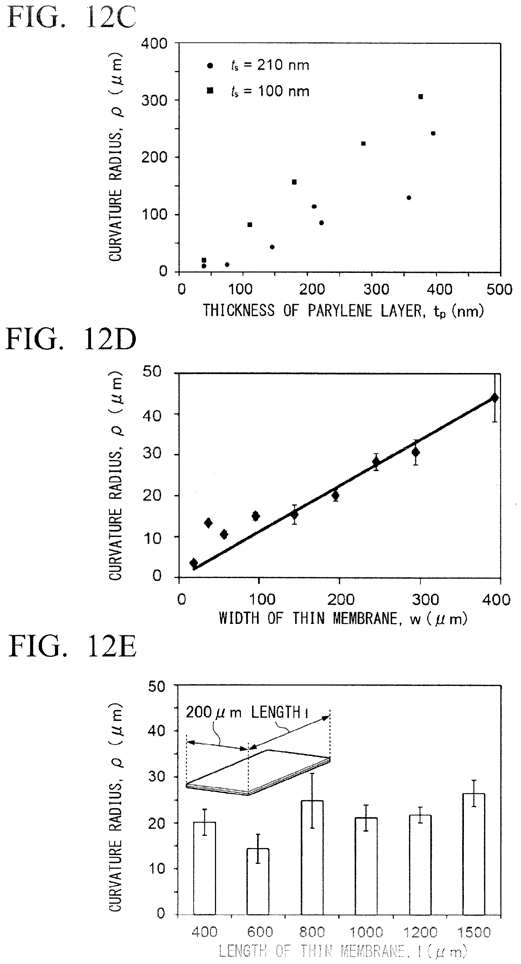

In Example 2, it was confirmed that the thin film 1 can be assembled into a three-dimensional shape in a self-assembling manner using the strain distribution due to buckling in the in-plane direction caused by the difference in mechanical strength between the thin film layer 10 and the thin film layer 11. Furthermore, it was found that the curvature radius of the tubular structure in a steady state after completion of self-assembly depends only on the ratio of thickness and the ratio of mechanical strength between the two thin film layers. FIGS. 12A to 12E show correlations among the curvature radius ρ of the thin film 1, and the thickness tₚ of the thin film layer 11 composed of parylene (FIGS. 12B, 12C), the lateral width w of the thin film 1 (FIG. 12D), and the length I of the thin film 1 in the major axis direction (FIG. 12E). When only the thickness tₚ of the thin film layer 11 among the two thin film layers constituting the thin film 1 was increased, the curvature radius ρ of the thin film 1 also increased accordingly (FIG. 12B). In addition, it was observed that when the thickness tₛ of the thin film layer 10 was reduced, the curvature radius ρ of the thin film 1 tended to increase accordingly (FIG. 12C). Furthermore, it was observed that when the thickness of the thin film 1 was made constant, in the rectangular thin film 1, the curvature radius ρ of the thin film 1 was almost linearly proportional to the length (width w) in the minor axis direction (FIG. 12D), whereas it was hardly affected by the length I in the major axis direction (FIG. 12E). In the thin film 1 composed of a silk fibroin gel layer serving as the thin film layer 10 and a parylene layer serving as the thin film layer 11, since the silk fibroin gel layer has an elastic modulus of 1 to 100 MPa and the parylene layer has an elastic modulus of 1 to 10 GPa, the ratio of the elastic moduli of the two layers (that is, (elastic modulus of the silk fibroin gel layer) / (elastic modulus of the parylene layer)) can be a value ranging from 0.0001 to 0.1. However, if there is a difference in elastic modulus between the two thin film layers, the ratio of the elastic moduli is not particularly limited. The method of measuring the elastic modulus is not particularly limited as long as the same measurement method is employed for the polymer material used for the thin film layer 10 and the polymer material used for the thin film layer 11. Examples of the methods of measuring the elastic modulus include, for example, methods described in Jiang and others (Jiang C et al., Adv. Funct. Mater. 2007, 17, 2229-2237) and Hu and others (Hu X et al., Biomacromolecules. 2011 May 9; 12 (5): 1686-96), and the like.

Since the tubular structure of the present example has mobility, it can be placed on a microelectrode array (MEA) substrate that measures existing extracellular potential by controlling the position with a capillary, and can be applied to highly efficient measurement of extracellular potential of any cell at any time.

### Industrial Applicability

According to the present invention, since cells are encapsulated in a thin film three-dimensional structure formed of a soft material exhibiting high biocompatibility, it becomes possible to produce biological devices and artificial tissues exhibiting high biocompatibility. The present invention can be used in the overall field of using biological tissue-like structures including regenerative medicine technology and drug screening. In addition, the present invention can also be applied to body implantable device elements and extracellular potential measuring elements.

### Reference Signs List

1: Thin film;
10: First thin film layer;
11: Second thin film layer;
12: Sacrificial layer;
13: Substrate;
20: Microparticle;
21: Modified protein layer;
30: Photoresist film

## Claims

1. A three-dimensional structure having an internal space, comprising:
a polymer film having a plurality of layers,
wherein a microparticle is encapsulated in the internal space of said three-dimensional structure,
the polymer film having the plurality of layers consists of a silk fibroin gel layer and a polyparaxylen layer, and
a layer in contact with an outside of the three-dimensional structure among the layers of the polymer film having the plurality of layers is composed of the silk fibroin gel layer.

2. The three-dimensional structure according to Claim 1, wherein the microparticle is a cell.

3. The three-dimensional structure according to Claim 1 or 2, further comprising a layer composed of an extracellular matrix on a surface of the polymer film.

4. The three-dimensional structure according to any one of Claims 1 to 3, wherein the polymer film has a thickness of 15 to 400 nm.

5. The three-dimensional structure according to any one of Claims 2 to 4, wherein the cell is an adherent cell and is adhered to the polymer film.

6. The three-dimensional structure according to any one of Claims 2 to 5, wherein the cells grow according to the shape of the internal space of the three-dimensional structure.

7. A biological tissue-like structure comprising:
the three-dimensional structure according to any one of Claims 2 to 6; and
a cell existing outside the three-dimensional structure,
wherein a cell encapsulated in the three-dimensional structure forms a structure extending to an outside of the three-dimensional structure, and
an intercellular interaction is able to occur between the cell encapsulated in the three-dimensional structure and the cell existing outside the three-dimensional structure.

8. A method for producing a three-dimensional structure encapsulating a microparticle,
the method comprising the steps of:
(a) forming a polymer film having a plurality of layers on a sacrificial layer formed on a substrate by forming a silk fibroin gel layer on the sacrificial layer and forming a polyparaxylen layer on the silk fibroin gel layer;
(b) floating the microparticle over a surface of the polymer film having the plurality of layers and
(c) decomposing the sacrificial layer, thereby releasing the polymer film having the plurality of layers from the substrate to form a three-dimensional structure in a self-assembling manner in the polymer film having the plurality of layers.

9. The method for producing a three-dimensional structure according to Claim 8, further comprising a step of forming the sacrificial layer on the substrate,
wherein the step (b) is a step of adding a suspension containing the microparticle to the polymer film having the plurality of layers.

10. The method for producing a three-dimensional structure according to Claim 8 or 9, wherein the microparticle is a cell.

11. The method for producing a three-dimensional structure according to any one of Claims 8 to 10, further comprising a step of forming a layer composed of an extracellular matrix on a surface of the polymer film having the plurality of layers.

12. The method for producing a three-dimensional structure according to any one of Claims 8 to 11, wherein the polymer film having the plurality of layers has a thickness of 15 to 400 nm.

13. A laminate comprising:
a substrate;
a sacrificial layer laminated on the substrate; and
a polymer film having a plurality of layers laminated on the sacrificial layer,
wherein the polymer film having the plurality of layers consists of a silk fibroin gel layer and a polyparaxylen layer, the silk fibroin gel layer is laminated on the sacrificial layer, and the polyparaxylen layer is laminated on the silk fibroin gel layer.

14. The laminate according to Claim 13, further comprising a layer composed of an extracellular matrix laminated on the polymer film having the plurality of layers.

15. The laminate according to Claim 13 or 14, wherein the polymer film having the plurality of layers has a thickness of 15 to 400 nm.

## Patentansprüche

1. Dreidimensionale Struktur mit einem internen Raum, umfassend:
einen Polymerfilm mit einer Mehrzahl von Schichten,
wobei ein Mikropartikel in dem internen Raum der dreidimensionalen Struktur eingekapselt ist,
wobei der Polymerfilm mit der Mehrzahl von Schichten aus einer Seidenfibroingelschicht und einer Polyparaxylenschicht besteht, und
wobei eine Schicht in Kontakt mit einer Außenseite der dreidimensionalen Struktur aus den Schichten des Polymerfilms mit der Mehrzahl von Schichten aus der Seidenfibroingelschicht gebildet ist.

2. Dreidimensionale Struktur nach Anspruch 1, wobei das Mikropartikel eine Zelle ist.

3. Dreidimensionale Struktur nach Anspruch 1 oder 2, ferner umfassend eine Schicht, die aus einer extrazellulären Matrix gebildet ist, auf einer Oberfläche des Polymerfilms.

4. Dreidimensionale Struktur nach einem der Ansprüche 1 bis 3, wobei der Polymerfilm eine Dicke von 15 bis 400 nm hat.

5. Dreidimensionale Struktur nach einem der Ansprüche 2 bis 4, wobei die Zelle eine adhärente Zelle ist und an dem Polymerfilm anhaftet.

6. Dreidimensionale Struktur nach einem der Ansprüche 2 bis 5, wobei die Zellen entsprechend der Gestalt des internen Raums der dreidimensionalen Struktur wachsen.

7. Struktur ähnlich einem biologischen Gewebe, umfassend:
die dreidimensionale Struktur nach einem der Ansprüche 2 bis 6; und
eine Zelle, die außerhalb der dreidimensionalen Struktur existiert,
wobei eine Zelle, die in der dreidimensionalen Struktur eingekapselt ist, eine Struktur bildet, die sich zu einer Außenseite der dreidimensionalen Struktur erstreckt, und
eine interzelluläre Interaktion zwischen der Zelle, die in der dreidimensionalen Struktur eingekapselt ist, und der Zelle auftreten kann, die außerhalb der dreidimensionalen Struktur existiert.

8. Verfahren zur Erzeugung einer dreidimensionalen Struktur, die ein Mikropartikel einkapselt,
wobei das Verfahren die folgenden Schritte umfasst:
(a) Bilden eines Polymerfilms mit einer Mehrzahl von Schichten auf einer Opferschicht, die auf einem Substrat gebildet ist, durch Bilden einer Seidenfibroingelschicht auf der Opferschicht und Bilden einer Polyparaxylenschicht auf der Seidenfibroingelschicht;
(b) Flotieren des Mikropartikels über eine Oberfläche des Polymerfilms mit der Mehrzahl von Schichten, und
(c) Zersetzen der Opferschicht, wodurch der Polymerfilm mit der Mehrzahl von Schichten von dem Substrat gelöst wird, um eine dreidimensionale Struktur in einer selbstorganisierenden Weise in dem Polymerfilm mit der Mehrzahl von Schichten zu bilden.

9. Verfahren zur Erzeugung einer dreidimensionalen Struktur nach Anspruch 8, ferner umfassend einen Schritt des Bildens der Opferschicht auf dem Substrat,
wobei der Schritt (b) ein Schritt des Hinzufügens einer Suspension, die das Mikropartikel enthält, zu dem Polymerfilm mit der Mehrzahl von Schichte ist.

10. Verfahren zur Erzeugung einer dreidimensionalen Struktur nach Anspruch 8 oder 9, wobei das Mikropartikel eine Zelle ist.

11. Verfahren zur Erzeugung einer dreidimensionalen Struktur nach einem der Ansprüche 8 bis 10, ferner umfassend einen Schritt des Bildens einer Schicht, die aus einer extrazellulären Matrix gebildet ist, auf einer Oberfläche des Polymerfilms mit der Mehrzahl von Schichten.

12. Verfahren zur Erzeugung einer dreidimensionalen Struktur nach einem der Ansprüche 8 bis 11, wobei der Polymerfilm mit der Mehrzahl von Schichten eine Dicke von 15 bis 400 nm hat.

13. Laminat, umfassend:
ein Substrat;
eine Opferschicht, die auf das Substrat laminiert ist; und
einen Polymerfilm mit einer Mehrzahl von Schichten, der auf die Opferschicht laminiert ist,
wobei der Polymerfilm mit der Mehrzahl von Schichten aus einer Seidenfibroingelschicht und einer Polyparaxylenschicht besteht, wobei die Seidenfibroingelschicht auf die Opferschicht laminiert ist, und wobei die Polyparaxylenschicht auf die Seidenfibroingelschicht laminiert ist.

14. Laminat nach Anspruch 13, ferner umfassend eine Schicht, die aus einer extrazellulären Matrix gebildet ist, die auf den Polymerfilm mit der Mehrzahl von Schichten laminiert ist.

15. Laminat nach Anspruch 13 oder 14, wobei der Polymerfilm mit der Mehrzahl von Schichten eine Dicke von 15 bis 400 nm hat.

## Revendications

1. Structure tridimensionnelle ayant un espace interne, comprenant :
un film polymère ayant une pluralité de couches,
dans laquelle une microparticule est encapsulée dans l'espace interne de ladite structure tridimensionnelle,
le film polymère ayant la pluralité de couches est constitué d'une couche de gel de fibroïne de soie et d'une couche de polyparaxylène, et
une couche en contact avec un extérieur de la structure tridimensionnelle parmi les couches du film polymère ayant la pluralité de couches est composée de la couche de gel de fibroïne de soie.

2. Structure tridimensionnelle selon la revendication 1, dans laquelle la microparticule est une cellule.

3. Structure tridimensionnelle selon la revendication 1 ou 2, comprenant en outre une couche composée d'une matrice extracellulaire sur une surface du film polymère.

4. Structure tridimensionnelle selon l'une quelconque des revendications 1 à 3, dans laquelle le film polymère a une épaisseur de 15 à 400 nm.

5. Structure tridimensionnelle selon l'une quelconque des revendications 2 à 4, dans laquelle la cellule est une cellule adhérente et est mise à adhérer au film polymère.

6. Structure tridimensionnelle selon l'une quelconque des revendications 2 à 5,
dans laquelle les cellules croissent selon la forme de l'espace interne de la structure tridimensionnelle.

7. Structure de type tissu biologique comprenant :
la structure tridimensionnelle selon l'une quelconque des revendications 2 à 6 ; et
une cellule existant à l'extérieur de la structure tridimensionnelle,
dans laquelle une cellule encapsulée dans la structure tridimensionnelle forme une structure s'étendant vers un extérieur de la structure tridimensionnelle, et
une interaction intercellulaire est capable de se produire entre la cellule encapsulée dans la structure tridimensionnelle et la cellule existant à l'extérieur de la structure tridimensionnelle.

8. Procédé de production d'une structure tridimensionnelle encapsulant une microparticule,
le procédé comprenant les étapes consistant à :
(a) former un film polymère ayant une pluralité de couches sur une couche sacrificielle formée sur un substrat par formation d'une couche de gel de fibroïne de soie sur la couche sacrificielle et formation d'une couche de polyparaxylène sur la couche de gel de fibroïne de soie ;
(b) mettre en flottement la microparticule sur une surface du film polymère ayant la pluralité de couches et
(c) décomposer la couche sacrificielle, pour ainsi libérer le film polymère ayant la pluralité de couches du substrat pour former une structure tridimensionnelle de manière auto-assemblée dans le film polymère ayant la pluralité de couches.

9. Procédé de production d'une structure tridimensionnelle selon la revendication 8, comprenant en outre une étape consistant à former la couche sacrificielle sur le substrat,
dans lequel l'étape (b) est une étape consistant à ajouter une suspension contenant la microparticule au film polymère ayant la pluralité de couches.

10. Procédé de production d'une structure tridimensionnelle selon la revendication 8 ou 9, dans lequel la microparticule est une cellule.

11. Procédé de production d'une structure tridimensionnelle selon l'une quelconque des revendications 8 à 10, comprenant en outre une étape consistant à former une couche composée d'une matrice extracellulaire sur une surface du film polymère ayant la pluralité de couches.

12. Procédé de production d'une structure tridimensionnelle selon l'une quelconque des revendications 8 à 11, dans lequel le film polymère ayant la pluralité de couches a une épaisseur de 15 à 400 nm.

13. Stratifié comprenant :
un substrat ;
une couche sacrificielle stratifiée sur le substrat ; et
un film polymère ayant une pluralité de couches stratifié sur la couche sacrificielle,
dans lequel le film polymère ayant la pluralité de couches est constitué d'une couche de gel de fibroïne de soie et d'une couche de polyparaxylène, la couche de gel de fibroïne de soie est stratifiée sur la couche sacrificielle, et la couche de polyparaxylène est stratifiée sur la couche de gel de fibroïne de soie.

14. Stratifié selon la revendication 13, comprenant en outre une couche composée d'une matrice extracellulaire stratifiée sur le film polymère ayant la pluralité de couches.

15. Stratifié selon la revendication 13 ou 14, dans lequel le film polymère ayant la pluralité de couches a une épaisseur de 15 à 400 nm.
